# EUROPEAN PATENT SPECIFICATION

(11) **EP 2 789 245 B1**
(45) Date of publication and mention of the grant of the patent: **17.01.2018**
(21) Application number: 12855854.1
(22) Date of filing: 23.04.2012
(51) Int. Cl.: A23L 29/10, A23L 29/25, A23L 2/52, A23L 2/56, A23L 2/58, A23L 27/00, A23D 7/00, A61K 8/06, A61K 8/73, A61Q 13/00

(54) **EMULSION COMPOSITION, AND COMPOSITION CONTAINING SAME**
EMULSIONSZUSAMMENSETZUNG UND DIESE ENTHALTENDE ZUSAMMENSETZUNG
COMPOSITION D'ÉMULSION ET COMPOSITION LA CONTENANT

(30) Priority: 09.12.2011 JP 2011270688
(43) Date of publication of application: 15.10.2014
(73) Proprietor: San-Ei Gen F.F.I., INC., Toyonaka-shi, Osaka 561-8588 (JP)
(72) Inventor: SAKATA, Makoto, Toyonaka-shi, Osaka 561-8588, (JP); NISHINO, Masayuki, Toyonaka-shi, Osaka 561-8588, (JP); NAKAO, Tomohiro, Toyonaka-shi, Osaka 561-8588, (JP); MIYAMOTO, Kanako, Toyonaka-shi, Osaka 561-8588, (JP)
(74) Representative: Müller-Boré & Partner Patentanwälte PartG mbB
(86) International application number: PCT/JP2012/060869
(87) International publication number: WO 2013/084518

(56) References cited:
- EP-A1- 2 008 532
- WO-A1-2006/030850
- JP-A- 2006 257 246
- JP-A- 2010 124 817
- JP-A- 2010 126 718
- JP-A- 2010 187 655
- JP-A- 2011 000 032
- JP-A- 2011 125 279
- JP-A- 2011 177 120
- KATAYAMA,T. ET AL.: 'Natural Hydrocolloid Emulsifiers (2) Characteristics of the Adsorbed Component of Gum Ghatti Responsible for Its Oil-Water Interface Advantages' FOODS & FOOD INGRED J JPN vol. 213, no. 4, 2008, pages 372 - 376, XP008169900
- IDO,T. ET AL.: 'Natural Hydrocolloid Emulsifiers (2) Emulsification Properties of GATIFOLIA (Gum Ghatti) Used for Emulsions in Food Products' FOODS & FOOD INGRED J JPN vol. 213, no. 4, 2008, pages 365 - 371, XP008169902
- RYO MARUYAMA ET AL.: 'FFI reports: Gum ghatti- type clear emulsified flavors' FOODS & FOOD INGRED J JPN vol. 214, no. 4, 2009, pages 516 - 518, XP008169901
- TAKAO IDO: 'FFI Reports Ghatti Gum' FOODS & FOOD INGRED J JPN vol. 211, no. 7, 2006, pages 641 - 645, XP008169899

## Description

The present invention relates to an emulsion composition. More specifically, the present invention relates to an emulsion composition that ensures excellent emulsion stability and excellent dispersibility and solubility in water, as well as excellent transparency and storage stability of a solution prepared by adding the emulsion composition to water.

In the food industry, for example, flavorings are widely used as additives for imparting various tastes and flavors to food. These flavorings are used by being processed into water-soluble or oil-soluble preparations depending on the food or beverages to which the flavor is imparted. Since it is difficult to directly add an oil-soluble flavoring component to aqueous food or beverages, an oil-soluble flavoring is usually processed into an oil-in-water emulsified flavoring by being dispersed in an aqueous solution using a protective colloid substance or an emulsifier.

Specific examples of emulsified flavorings include flavorings obtained by emulsifying oil-soluble flavorings using a chemically synthesized surfactant, such as glycerin fatty acid ester, sucrose fatty acid ester, or polysorbate, and/or a surfactant derived from natural products such as lecithin, enzymatically decomposed lecithin, or saponin, thereby enabling it to be transparently dissolved in water; and flavorings obtained by emulsifying an oil phase component containing an oil-soluble flavoring and a specific-gravity-adjusting agent in an aqueous solution using a protective colloid substance (for example, gum arabic, modified starch, dietary soy fiber, or the like), thereby expressing turbidity similar to a turbid fruit juice and a flavor peculiar to an oil-soluble flavoring when it is incorporated in a beverage.

However, although the emulsified flavoring prepared by using a surfactant can be transparently dissolved in water, the emulsified flavoring by itself is susceptible to time-dependent change (insufficient storage stability), and also is inferior in emulsion stability when it is added to an aqueous food or beverage. Therefore, these emulsified flavorings have problems including a time-dependent decrease in transparency of the emulsified flavoring by itself, a decrease in transparency when it is added to an aqueous food or beverage, and the taste peculiar to a surfactant that affects the taste of the final food product. Because of such problems, the oil-soluble flavorings have been used only for limited types of food.

Further, the turbid-dissolution-type emulsified flavoring obtained by using a protective colloid substance or the like generates turbidity in the food, and thus can be used only for limited types of food. Further, although this flavoring has improved flavor sustainability due to the emulsification using a protective colloid substance, the original flavor of the oil-soluble flavoring cannot be exhibited; thus, the turbid-solution emulsified flavoring has a problem of insufficient flavor expression compared with a water-soluble flavoring.

To solve such problems, there have been repeated studies of various modifications of flavoring preparations, which are used as additives, in order to improve flavor expression.

Specifically, studies have disclosed examples such as a beverage containing an emulsion composition obtained by mixing an emulsified flavoring and a stable emulsified flavor composition at a ratio of 3:7 to 7:3 (Patent Document 1); a structure in which an oil-soluble compound is coated with an emulsifier to be dispersed in a sugar alcohol, the structure being obtained by heating and melting a sugar alcohol composition containing an oil-soluble compound and an emulsifier and keeping the molten matter at a temperature of 5 to 40°C (Patent Document 2); a flavoring produced by extracting a recovered aroma obtained by gas-liquid countercurrent catalytic extraction of a plant-derived material using vegetable oils and fats, animal oils and fats, hardened oils thereof, middle chain fatty acid triglycerides, and the like (Patent Document 3); and a beverage product comprising an oil-in-water beverage emulsion which contains an unweighted oil, water, and a food grade stabilizer (Patent Document 4).

However, these methods have a problem in that such attempts to improve the expression of the flavor requires additional production steps which are not performed in the hitherto-known methods for producing a flavoring preparation.

Further, other documents have disclosed a method using a specific polyglycerin fatty acid ester (Patent Documents 5 and 6), and a method of specifying the proportion of an oil-soluble component in an oil-soluble flavoring and dispersing or emulsifying it in a solution containing a specific surfactant such as gum ghatti or gum arabic (Patent Document 7), as a method for stabilizing an emulsified flavoring.

Patent Document 1: JP2004-168909A
Patent Document 2: JP2004-33820A
Patent Document 3: JP2002-105485A
Patent Document 4: JPH11-509421A
Patent Document 5: JP2006-50986A
Patent Document 6: JP2007-267683A
Patent Document 7: JP2006-257246A

JP2010-124817A describes an emulsified composition for beverages which is capable of stably dispersing and holding a fat-soluble ingredient in the beverages at a high concentration, and further imparting moderate turbidity. JP2011-32A describes an emulsion composition containing hop extract. EP 2 008 532 A1 describes an emulsion composition comprising a capsinoid compound with an improved dilution stability thereof.

An object of the present invention is to provide an emulsion composition having excellent emulsion stability and excellent dispersibility or solubility in water, as well as excellent transparency and storage stability of a solution prepared by adding the emulsion composition to water. More specifically, an object of the present invention is to provide an emulsion composition that has excellent emulsion stability by itself, as well as excellent dispersibility or solubility in water when it is dispersed or dissolved in water, thereby producing an aqueous solution containing an emulsified preparation having excellent transparency (low turbidity) and excellent storage stability.

Another object of the present invention is to provide a method for preparing a composition, in particular, a solution composition (liquid composition), preferably a food or a beverage, which is prepared by using the emulsion composition.

The inventors of the present invention conducted extensive research to solve the above problems by focusing attention on the emulsion stability of emulsion compositions (for example, emulsified flavorings or emulsified colorants), dispersibility or solubility in water of emulsion compositions, and transparency and storage stability of a liquid composition obtained by adding an emulsion composition to water. As a result of the research, the inventors found that a desired emulsion composition can be obtained by emulsifying an oil phase component and an aqueous phase component using, as an emulsifier, gum ghatti that has a viscosity in a range of 50 to 3000 mPa·s under a predetermined condition (20ºC, 30 rpm), in a proportion of more than 25 parts by weight based on 100 parts by weight of the oil phase component, wherein the proportion of gum ghatti in the aqueous phase component containing the gum ghatti is 1 to 6 wt% based on 100 wt% of the total amount of the aqueous phase component containing the gum ghatti.

More specifically, the emulsion composition having the above formulation has excellent emulsion stability by itself, and also can ensure excellent storage stability when it is dispersed or dissolved in water. As shown in the Experiment Examples, it was confirmed that coarsening due to the aggregation of emulsified particles did not easily occur even when the emulsion composition was shaken, and the generation of insoluble matter by the deterioration of particles was suppressed; thus, high transparency was stably maintained.

The present invention was completed based on these findings and encompasses the following aspects.

### (I) Emulsion Composition

(I-1). An emulsion composition prepared by emulsifying an oil phase component and an aqueous phase component using gum ghatti, wherein the emulsion composition comprises gum ghatti in a proportion of more than 25 parts by weight based on 100 parts by weight of the oil phase component; the gum ghatti has a viscosity of 50 to 3000 mPa·s, which is measured by preparing a 15 wt% aqueous solution of the gum ghatti and measuring its viscosity for 1 minute at 20°C and 30 rpm using a Brookfield viscometer; and
   the absorbency (1%E) (cell width = 1 cm) of an aqueous solution obtained by diluting the emulsion composition immediately after preparation with water at a concentration of 1 wt% is less than 0.5, which is measured at a wavelength of 720 nm in comparison with ion exchange water,
   and wherein the proportion of gum ghatti in the aqueous phase component containing the gum ghatti is 1 to 6 wt% based on 100 wt% of the total amount of the aqueous phase component containing the gum ghatti.
(I-2). The emulsion composition according to (I-1), wherein the oil phase component comprises at least one member selected from the group consisting of oil-soluble flavorings, oil-soluble colorants, and oil-soluble bioactive substances,
(I-3). The emulsion composition according to (1-2), wherein the oil-soluble bioactive substance is at least one member selected from the group consisting of a fat-soluble vitamins, docosahexaenoic acid, eicosapentaenoic acid, coenzyme Q₁₀, α-lipoic acids, α-linolenic acid, oil-soluble polyphenols, sesamin, phytosterols, and glycosyl ceramides.
(I-4). The emulsion composition according to any one of (I-1) to (1-3), wherein the oil phase component comprises at least one member selected from the group consisting of glycerin fatty acid esters, medium-chain triglycerides, sucrose acetate isobutyrate, and vegetable oils or fats, more preferably at least one member selected from the group consisting of glycerin fatty acid esters and medium-chain triglycerides.
(1-5). The emulsion composition according to (I-4), wherein the glycerin fatty acid ester is a polyglycerin fatty acid ester in which 5 to 8 molecules of C₂₋₁₀ saturated fatty acid are bonded to polyglycerin having an average polymerization degree of 3 to 10 by ester bonds.
(I-6). The emulsion composition according to (1-4) or (1-5), wherein the oil phase component further comprises lecithin.
(I-7). The emulsion composition according to any one of (I-1) to (1-6), wherein the emulsion composition is at least one member selected from the group consisting of emulsified flavoring preparations, emulsified colorant preparations, and emulsified functional preparations.
(1-8). The emulsion composition according to any one of (I-1) to (1-7), wherein the emulsion composition is an O/W type emulsion.

### (II) Method for Preparing Emulsion Composition

(II-1). A method for preparing an emulsion composition, comprising the step of emulsifying an oil phase component and an aqueous phase component using gum ghatti, wherein the emulsion composition comprises gum ghatti in a proportion of more than 25 parts by weight based on 100 parts by weight of the oil phase component.; and the gum ghatti has a viscosity of 50 to 3000 mPa·s, which is measured by preparing a 15 wt% aqueous solution of the gum ghatti and measuring its viscosity for 1 minute at 20°C and 30 rpm using a Brookfield viscometer, and
   the absorbency (1%E) (cell width = 1 cm) of an aqueous solution obtained by diluting the emulsion composition immediately after preparation with water at a concentration of 1 wt% is less than 0.5, which is measured at a wavelength of 720 nm in comparison with ion exchange water,
   and wherein the proportion of gum ghatti in the aqueous phase component containing the gum ghatti is 1 to 6 wt% based on 100 wt% of the total amount of the aqueous phase component containing the gum ghatti.
(II-2). The method according to (II-1), wherein the oil phase component comprises at least one member selected from the group consisting of oil-soluble flavorings, oil-soluble colorants, and oil-soluble bioactive substances.
(II-3). The method according to (II-2), wherein the oil-soluble bioactive substance is at least one member selected from the group consisting of fat-soluble vitamins, docosahexaenoic acid, eicosapentaenoic acid, coenzyme Q₁₀, α-lipoic acids, α-linolenic acid, oil-soluble polyphenols, sesamin, phytosterols, and glycosyl ceramides.
(II-4). The method according to any one of (II-1) to (II-3), wherein, the oil phase component comprises at least one member selected from the group consisting of glycerin fatty acid esters, medium-chain triglycerides, sucrose acetate isobutyrate, and vegetable oils or fats, more preferably at least one member selected from the group consisting of glycerin fatty acid esters and medium-chain triglycerides.
(II-5). The method according to (II-4), wherein the glycerin fatty acid ester is a polyglycerin fatty acid esters in which 5 to 8 molecules of C₂₋₁₀ saturated fatty acid are bonded to polyglycerin having an average polymerization degree of 3 to 10 by ester bonds.
(II-6). The method according to (II-4) or (II-5), wherein the oil phase component further comprises lecithin.
(II-7). The method according to any one of (II-1) to (II-6), wherein the emulsion composition is at least one member selected from the group consisting of emulsified flavoring preparations, emulsified colorant preparations, and emulsified functional preparations.
(II-8). The method according to any one of (II-1) to (II-7), wherein the emulsion composition is an O/W emulsion.

### (IV) Method for Preparing the Composition

(IV-1). A method for preparing a composition selected from the group consisting of food, beverages, fragrances, cosmetics, pharmaceuticals and quasi-drugs, the method comprising the step of dissolving or dispersing the emulsion composition of any one of (1-1) to (1-8) in an aqueous solvent.
(IV-2). The method according to (IV-1), wherein the composition is a liquid.
(IV-3). The method according to (IV-1) or (IV-2), wherein the composition is a food, a beverage, a cosmetic, a pharmaceutical, or a quasi-drug.
(IV-4). The method according to (IV-1) or (IV-2), wherein the composition is a food or a beverage.

The emulsion composition of the present invention has excellent emulsion stability and can thus be provided as an emulsified preparation with high storage stability. Further, since the emulsion composition of the present invention has high solubility in an aqueous solvent, it is possible to prepare a composition (water-containing composition, in particular, aqueous solution composition) with high transparency by using the emulsion composition of the present invention. Further, the emulsion composition of the present invention has not only high dispersibility or solubility in an aqueous solvent, but also excellent stability in an aqueous solvent; thus, coarsening of the emulsified particles does not easily occur due to shaking during the storage of the emulsion compositions. Therefore, turbidity or sedimentation can be suppressed for a long period of time. With such characteristics, the emulsion composition of the present invention is useful for the preparation of a water-containing composition, in particular, an aqueous solution composition, that requires lasting transparency.

### (I) Emulsion Composition and Method for Preparing the Composition

The emulsion composition of the present invention is a composition obtained by emulsifying an oil phase component and an aqueous phase component using, as an emulsifier, gum ghatti that has a viscosity in a range of 50 to 3000 mPa·s under a predetermined condition (20ºC, 30 rpm), in a proportion of more than 25 parts by weight based on 100 parts by weight of the oil phase component. In other words, the emulsion composition of the present invention can be prepared by emulsifying an oil phase component and an aqueous phase component using, as an emulsifier, gum ghatti having the above range of viscosity in a proportion of more than 25 parts by weight based on 100 parts by weight of the oil phase component.

### (1) Oil Phase Component (Oil-soluble Component or Fat-soluble Component for Constituting an Oil Phase)

In the emulsion composition, the oil phase component (oil-soluble component or fat-soluble component contained in the oil phase) contains an oil-soluble material (including a fat-soluble material; the same applies hereafter) and an oil-based solvent for dissolving the oil-soluble material.

### (1-1) Oil-soluble Material

Examples of the oil-soluble material include, but are not limited to, oil-soluble flavorings, oil-soluble colorants, and oil-soluble bioactive substances.

### (1-1-1) Oil-soluble Flavoring

The oil-soluble flavoring (including a fat-soluble flavoring; the same applies hereafter) used in the present invention is not limited insofar as it is an oil-soluble or a fat-soluble substance containing an aroma component. Preferably, the oil-soluble flavoring is an edible flavoring that can be incorporated in a food or a beverage, or a fragrance that can be used on the human body as a cosmetic material.

Examples include various extracts from natural materials, including animal-derived or plant-derived materials, obtained through non-volatile solvent extraction, volatile solvent extraction, or supercritical extraction; natural flavorings such as essential oils or recovered flavors obtained by steam distillation or compression; synthetic flavorings produced by chemical synthesis methods; and flavoring bases obtained by incorporating/dissolving these flavorings in oils or solvents. Examples of natural flavorings include extracts such as absolutes, extracts, or oleoresins; essential oils such as cold press oils; and alcohol extracts such as tinctures.

Specific examples of these flavorings include essential citrus oils such as orange oil, lemon oil, grapefruit oil, lime oil, or mandarin oil; essential flower oils or absolutes such as lavender oil; essential oils such as peppermint oil, spearmint oil, or cinnamon oil; essential oils or oleoresins of spices such as allspice, anise seed, basil, laurel, cardamom, celery, cloves, garlic, ginger, mustard, onion, paprika, parsley, or black pepper; synthetic flavorings such as limonene, linalool, geraniol, menthol, eugenol or vanillin; extracted oils from beans such as coffee, cacao, vanilla, or roasted peanuts; extracts from teas such as black tea, green tea, or oolong tea; and other synthetic flavoring compounds. Although these flavorings may be used solely, they are generally used as a mixed flavoring by combining any two or more kinds of them. The definition of "flavoring" used in the present invention includes not only flavorings made of a single compound but also such mixed flavorings.

### (1-1-2) Oil-soluble Colorant

The oil-soluble colorant (including a fat-soluble colorant; the same applies hereafter) used in the present invention is not limited insofar as it is an oil-soluble or a fat-soluble substance containing a colorant component. Preferably, the oil-soluble colorant is an edible colorant that can be incorporated in a food or a beverage, or a colorant that can be used on the human body as a cosmetic material.

Examples of such an oil-soluble colorant include paprika pigment, annatto pigment, tomato pigment, marigold pigment, *Haematococcus* pigment, *Dunaliella* carotene, carrot carotene, palm oil carotene, β-carotene, astaxanthin, canthaxanthin, lycopene, lutein, apocarotenal, fucoxanthin, cryptoxanthin, zeaxanthin, capsanthin, capsorubin, norbixin, bixin and chlorophyll. These oil-soluble colorants may be used solely or in a combination of two or more kinds.

### (1-1-3) Oil-soluble Bioactive Substance

The oil-soluble bioactive substance (including a fat-soluble bioactive substance; the same applies hereafter) used in the present invention is not limited insofar as it is an oil-soluble or a fat-soluble substance having an advantageous effect on living organisms. Preferably, the oil-soluble bioactive substance is an edible bioactive substance that can be incorporated in a food or a beverage, or a bioactive substance that can be used on the human body as a cosmetic material.

Examples of such an oil-soluble bioactive substance include oil-soluble pharmaceuticals; fat-soluble vitamins such as cod-liver oil, vitamin A (retinol, etc.), vitamin A oil, vitamin D (ergocalciferol, cholecalciferol, etc.), vitamin B₂ tetrabutyrate, ester of ascorbic acid and fatty acid, vitamin E (tocopherol, tocotrienol, etc.), or vitamin K (phylloquinone, menaquinone, etc.); plant-derived essential oils such as limonene, linalool, nerol, citronellol, geraniol, citral, 1-menthol, eugenol, cinnamic aldehyde, anethole, perillaldehyde, vanillin, or γ-undecalactone; resveratrol, oil-soluble polyphenols, glycosylceramide, sesamin, phosphatidylserine, coenzyme Q₁₀, ubiquinol, or α-lipoic acid; omega-3 fatty acids such as α-linolenic acid, eicosapentaenoic acid, or docosahexaenoic acid; omega-6 fatty acids such as linoleic acid or γ-linolenic acid; and bioactive components such as phytosterol. Among these, fat-soluble vitamins, coenzyme Q₁₀, α-lipoic acid, and omega-3 fatty acids such as α-linolenic acid, docosahexaenoic acid, or eicosapentaenoic acid are particularly preferable.

These oil-soluble bioactive substances may be used solely or in a combination of two or more kinds.

### (1-2) Oil-based Solvent

The oil-based solvent is not limited insofar as it can be used as a solvent for dissolving the above oil-soluble material, more specifically, insofar as it has compatibility with the above oil-soluble material. Preferably, the oil-based solvent is an edible substance that can be incorporated in a food or a beverage, or a substance that can be used on the human body as a cosmetic material.

Examples of such an oil-based solvent include vegetable oils and fats such as rapeseed oil, palm oil, soybean oil, olive oil, jojoba oil, coconut oil, gum elemi, or mastic resin; animal oils such as beef tallow or lard; sucrose acetate isobutyrate (SAIB), rosin, dammar gum, ester gum, glycerine fatty acid ester, and medium-chain triglyceride (MCT). These solvents may be used solely or in a combination of two or more kinds.

Preferable examples of oil-based solvents include glycerin fatty acid ester, medium-chain triglycerides, sucrose acetate isobutyrate, and vegetable oils and fats, more preferably glycerin fatty acid ester and medium-chain triglycerides.

Medium-chain triglyceride (MCT) herein refers to triacylglycerol constituted of medium chain fatty acids each having about 6 to 12, preferably 6 to 10, more preferably 8 to 10 carbon atoms. Any commercially available medium-chain triglycerides can be used, with no particular restriction. Examples include caprylic acid triglycerides, capric acid triglycerides, caprylic/capric triglycerides, and mixtures thereof.

Examples of glycerin fatty acid esters include polyglycerin fatty acid esters in which 5 to 8 molecules of C₂₋₁₀ saturated fatty acid are bonded to polyglycerin having an average polymerization degree of 3 to 10 by ester bonds. The average polymerization degree of the polyglycerin of the glycerin fatty acid ester is preferably 3 to 6. Further, preferable examples of the fatty acids bonded to the polyglycerin by ester bonds include a saturated fatty acid having 6 to 10, more preferably 8 to 10 carbon atoms. The glycerin fatty acid ester of the present invention may have a single ester component, or a mixture of a plurality of ester components.

Any commercially available glycerin fatty acid esters can be used with no particular restriction. Examples include Salacos HG-8 (Nisshin Oillio Group, Ltd.).

Further, as necessary, it is possible to add lecithin to the oil-based solvent. By adding lecithin, the average particle diameter of the emulsified particles contained in the emulsion composition decreases, thereby improving the emulsifying property. Further, addition of lecithin also makes it possible to prepare an emulsion composition with excellent transparency and storage stability when the emulsion composition is dissolved or dispersed in water.

"Lecithin" herein refers to a fat-soluble component having a phospholipid as a major component. The source of the lecithin is not particularly limited; examples include plant derived-lecithins obtained from oilseeds (such as soybean or rapeseed) and animal-derived lecithins obtained from egg yolk or the like. Preferably, the lecithin is an edible lecithin that can be incorporated in a food or a beverage, or a lecithin that can be used on the human body as a cosmetic material. Further, the lecithins usable in the present invention include modified lecithins such as fractionated lecithin, enzymatically decomposed lecithin, or enzymatically treated lecithin. These lecithins including modified lecithins may be obtained from marketed products, such as SLP-White (Tsuji Oil Mills Co., Ltd.).

The proportion of lecithin in the oil phase component is preferably 0.5 to 50 wt%, more preferably 4 to 20 wt%, per 100 wt% of the oil phase component. Further, the proportion of lecithin is preferably 0.01 to 5 wt%, more preferably 0.05 to 1 wt%, further preferably 0.1 to 0.5 wt%, per 100 wt% of the emulsion composition. In the present invention, the above oil-soluble material is mixed with an oil-based solvent so as to dissolve the oil-soluble material in the oil-based solvent, thus obtaining an oil phase component. The oil phase component is then dispersed or emulsified in an aqueous solution containing, as an emulsifier, a predetermined gum ghatti, thereby preparing an emulsion composition.

The mixing ratio (weight ratio) of the oil-soluble material to the oil-based solvent is generally, but not limited to, oil-soluble material:oil-based solvent = 80:20 to 20:80, preferably 40:60 to 20:80.

### (2) Emulsifier (Gum Ghatti) and Aqueous Phase Component Containing the Emulsifier (Emulsifier Solution)

As described above, the feature of the present invention is the use of a predetermined gum ghatti as an emulsifier.

Gum ghatti is a gum substance containing, as a major component, polysaccharide obtained by drying the trunk sap of *Anogeissus latifolia* Wall. Gum ghatti is publicly known as a thickening stabilizer (food additive).

The gum ghatti used in the present invention has a viscosity of 50 to 3000 mPa·s, preferably 50 to 2500 mPa·s, more preferably 50 to 1500 mPa·s, further more preferably 100 to 700 mPa·s, when the gum ghatti is prepared into an aqueous solution (20°C) having a concentration of 15 wt% and its viscosity is measured under the following conditions.

### ▪ Method for Measuring Viscosity

A 15 wt% gum ghatti aqueous solution was prepared to have a constant temperature (20°C), and the viscosity of the gum ghatti aqueous solution was measured by rotating it with a Brookfield rotational viscometer (Tokyo Keiki Inc., BM Model) for one minute at a rotation speed of 30 rpm. For this measurement, rotor No.2 is used to measure a viscosity of 50 to 500 mPa·s, and rotor No.3 is used to measure a viscosity of 500 to 4000 m Pa·s.

Such gum ghatti can be obtained from marketed products, such as "gum ghatti SD" (San-Ei Gen F.F.I., Inc.).

Upon preparation of the emulsion composition, the emulsifier is used in the form of an emulsifier solution by being dissolved or dispersed in an appropriate solvent, preferably, in an aqueous solvent.

Since the aqueous phase component (the water-soluble component contained in the aqueous phase) of the emulsion composition of the present invention is formed of a water-soluble material and an aqueous solvent for dissolving the water-soluble material, the aqueous solvent used herein to dissolve or disperse the emulsifier constitutes the aqueous phase of the emulsion composition of the present invention. Therefore, in the present invention, the "emulsifier solution" obtained by dissolving or dispersing the emulsifier in an aqueous solvent refers to a part or the whole of the aqueous phase component of the emulsion composition of the present invention. Examples of water-soluble materials include, but are not limited to, water-soluble vitamins (such as vitamin C), polysaccharide thickeners, antioxidant, chelating agents, pH regulators, and excipients (agents used for powderization, such as dextrin).

The aqueous solvents that can be used to prepare the emulsifier solution may be any solvent insofar as it has compatibility with gum ghatti; however, the aqueous solvent is preferably a solvent that can be incorporated in a food or a beverage, or a solvent that can be used on the human body as a cosmetic material. Examples of such solvents include water and polyhydric alcohols. These solvents may be used solely or in any combination of two or more. As described above, the solvent is preferably water, or an aqueous solvent obtained by mixing water and a polyhydric alcohol. By combining water and a polyhydric alcohol, it is possible to improve the stability of the emulsion composition, and the flavor expression of the emulsion composition.

Examples of polyhydric alcohols used in the present invention include glycerin, diglycerin, triglycerin, polyglycerin, propylene glycol, dipropylene glycol, 1,3-butylene glycol, ethylene glycol, polyethylene glycol, sorbitol (D-sorbitol), xylitol, maltitol, erythritol, mannitol, xylose, glucose, lactose, mannose, oligotose, high-fructose corn syrup, and sucrose. They may be used solely or in any combination of two or more kinds.

When a polyhydric alcohol is combined with water, the proportion of the polyhydric alcohol is, for example, 1 to 90 parts by weight, per 100 parts by weight of water.

The proportion of gum ghatti in the aqueous phase component containing the gum ghatti is 1 to 6 wt%, preferably 1 to 4 wt%, based on 100 wt%, i.e., the total amount, of the aqueous phase component containing the gum ghatti.

In the present invention, so as to ensure the emulsion stability of the emulsion composition, and the storage stability when the emulsion composition is dissolved or dispersed in water, the proportion of gum ghatti in the emulsion composition is more than 25 parts by weight, preferably at least 50 parts by weight, based on 100 parts by weight of the oil phase component. In view of the purpose and the effect of the present invention, there is no particular limitation of the upper limit of the proportion of gum ghatti; however, in view of the production costs, the proportion of gum ghatti is typically about 300 parts by weight.

### (3) Emulsion Composition and Method for Preparing Emulsion Composition

By mixing the oil phase component obtained above containing a mixture of an oil-soluble material and an oil-based solvent with an aqueous phase component containing an emulsifier solution, it is possible to obtain the emulsion composition of the present invention.

In addition to the oil phase component and the aqueous phase component containing an emulsifier solution, the emulsion composition of the present invention may also contain a water-soluble vitamin, a polysaccharide thickener, an antioxidant, a chelating agent, an oxidation inhibitor and the like insofar as the effects of the present invention are not impaired. When these components are oil-soluble (fat-soluble) materials, it is possible to incorporate them in the oil phase component. When these components are water-soluble materials, it is possible to incorporate them in the aqueous phase component.

The preparation of the emulsion composition may be performed using a commonly used technique for preparing an emulsified preparation. Examples include a method of stirring and mixing an oil phase component and an aqueous phase component containing an emulsifier solution using a homomixer, a colloid mill, a high-pressure homogenizer, an ultra-high-pressure homogenizer, a collision-type ultra-high-pressure homogenizer and the like. The stirring and mixing during the emulsification may be performed while heating or warming the mixture.

The proportion of the oil-soluble material in the thus-obtained emulsion composition may be appropriately changed according to the type of the oil-soluble material to be used or the type of the emulsion composition. For example, the proportion of the oil-soluble material is typically in a range of 0.1 to 5 wt%, preferably 1 to 4 wt%, more preferably 2 to 3 wt%, per 100 wt% of the emulsion composition.

The form of the emulsion composition of the present invention is not particularly limited. For example, the emulsion composition of the present invention may be prepared as an emulsion, or as a solid such as powder that is produced through spray drying, freeze-drying or like standard methods after adding an appropriate carrier or the like. The emulsion or the powder may be encapsulated in a capsule, such as a soft capsule or a hard capsule.

The emulsion composition thus prepared may be provided as various emulsified preparations according to the type of the oil-soluble material. For example, the emulsion composition may be used as an emulsified flavoring preparation when the emulsion composition contains an oil-soluble flavoring as the oil-soluble material, the emulsion composition may be used as an emulsified colorant preparation when the emulsion composition contains an oil-soluble colorant as the oil-soluble material, and the emulsion composition may be used as an emulsified functional preparation when the emulsion composition contains an oil-soluble bioactive substance as the oil-soluble material.

These emulsion compositions of the present invention have excellent emulsion stability by themselves, and also have excellent solubility in water; thus, the emulsion compositions of the present invention ensure excellent transparency and storage stability of water-containing products to which the emulsion compositions are added, such as food, beverages, fragrances, cosmetics, pharmaceuticals, or quasi-drugs.

Accordingly, when the emulsion composition of the present invention is prepared as, for example, an emulsified flavoring preparation, the emulsion composition may be used to prepare a water-containing flavored composition. When the emulsion composition of the present invention is prepared as an emulsified colorant preparation, the emulsion composition may be used to prepare a water-containing colored composition. When the emulsion composition of the present invention is prepared as an emulsified functional preparation, the emulsion composition may be used to prepare a water-containing functional composition. The flavored composition, colored composition, and functional composition may be food, beverages, fragrances, cosmetics, pharmaceuticals, or quasi-drugs.

### (II) Composition

By dispersing or dissolving the above emulsion composition in an aqueous solvent, it is possible to prepare a composition in which the emulsion composition is stably dispersed or dissolved in water. Examples of the aqueous solvents herein include water and solvents that have compatibility with water. Examples of the solvents that have compatibility with water include lower alcohols having 1 to 6, preferably 1 to 4, carbon atoms, and the above polyhydric alcohols. Preferable examples of lower alcohols include ethanol and propyl alcohol, more preferably ethanol.

The "composition" includes an aqueous composition prepared by a step of dispersing or dissolving the above emulsion composition of the present invention in an aqueous solvent. Examples of such a composition include a composition obtained by dispersing or dissolving the emulsion composition of the present invention in an aqueous solvent. This composition is generally in the form of a liquid (including a solution, an emulsified liquid, and a dispersion) or a semi-solid (including a paste and a cream). The examples of the forms of the composition further include a composition prepared by first dispersing or dissolving the above emulsion composition of the present invention in an aqueous solvent and then reducing or removing the aqueous solvent by a usual method such as distillation or drying. The emulsion composition thus prepared has a semi-solid form such as a paste, or a solid form such as powder or granules.

Examples of the compositions include flavored compositions, colored compositions, and functional compositions.

### (1) Flavored Composition

The flavored compositions include food, beverages, alcoholic food and beverages, pharmaceuticals, quasi-drugs, fragrances and cosmetics.

Examples of food, beverages, and alcoholic food and beverages include, but are not limited to, various beverages such as milk beverages, lactobacillus beverages, carbonated beverages, fruit-containing beverages (fruit-juice-containing beverages, fruit-juice-containing soft drinks, fruit-juice-containing carbonated beverages, fruit-pulp-containing beverages), vegetable-containing drinks, vegetable/fruit-containing drinks, alcoholic beverages such as liqueur, coffee drinks, powdered drinks, sport drinks, or nutritional supplement drinks, tea beverages such as black tea beverages, green tea beverages, or blended tea beverages; puddings such as custard pudding, milk pudding, or fruit-juice-containing pudding; desserts such as jellies, Bavarian cream, or yogurt; frozen desserts such as milk ice cream, fruit-juice-containing ice cream, soft-serve ice cream, ice lollipops; gum (stick gum and sugar-coated gum granules) such as chewing gum, or bubble gum; chocolates such as marble chocolate and other such coated chocolates, strawberry chocolate, blueberry chocolate, melon chocolate, and other flavored chocolates; candies such as hard candies (including bonbons, butterballs, and marbles), soft candies (including caramel, nougat, gummy candy, and marshmallow), drops, or taffy; soups such as consommé soup or potage soup; sauces such as vinaigrette dressings, non-oil dressings, ketchup, gravy, or sause; jams such as strawberry jam, blueberry jam, marmalade, apple jam, apricot jam, or preserves; fruit liquors such as red wine; and processed fruits such as candied cherries, apricots, apples, strawberries, or peaches. Among these, beverages, liquors, and jellies are preferable.

Examples of pharmaceuticals and quasi-drugs include syrup preparations, nutritional supplement drinks, tablets, capsules, tinctures, creams, and ointments. Nutritional supplement drinks and syrup preparations are preferable.

Examples of fragrances and cosmetics include tooth pastes, shampoos, hair conditioners, body soaps, and cosmetics.

The flavored composition may be produced through a production process commonly used for the production of various flavored products, except that it is necessary to incorporate the emulsified flavoring composition of the present invention as one of the materials in any step of the manufacture. Therefore, a special production device or a specific condition is not necessary to carry out the present invention; thus, the present invention is also industrially advantageous.

The amount of the emulsified flavoring composition of the present invention to be added to the composition, for which the flavor is to be added, is not particularly limited insofar as it is within a general amount of a flavoring for imparting a desired taste/flavor to a flavored product. For example, the amount of the emulsified flavoring composition of the present invention is about 0.01 to 50 wt%, preferably 0.05 to 20 wt%, more preferably about 0.1 to 10 wt%, per 100 wt% of the flavored composition.

### (2) Colored Composition

Examples of the colored composition include, as in the above flavored composition, food or beverages, alcohol-containing food or beverages, pharmaceuticals, quasi-drugs, and fragrances and cosmetics.

The colored composition of the present invention may be produced through a production process commonly used for the production of various colored products, except that it is necessary to incorporate the emulsified colorant composition of the present invention as one of the materials in any step of the manufacture. Therefore, a special production device or a specific condition is not necessary to carry out the present invention; thus, the present invention is also industrially advantageous.

The amount of the emulsified colorant composition of the present invention to be added to the composition, for which the colorant is to be added, is not particularly limited insofar as it is within a general amount of a colorant for adding a desired color to a colored product. For example, the amount of the emulsified colorant composition of the present invention is about 0.01 to 50 wt%, preferably 0.05 to 20 wt%, more preferably about 0.1 to 10 wt%, per 100 wt% of the colored composition.

### (3) Functional Composition

Examples of the functional composition include, as in the above flavoring or colorant composition, food or beverages, alcohol-containing food or beverages, pharmaceuticals, quasi-drugs, and fragrances and cosmetics.

The functional composition may be produced through a production process commonly used for the production of various functional products, except that it is necessary to incorporate the emulsified functional composition of the present invention as one of the materials in any step of the manufacture. Therefore, a special production device or a specific condition is not necessary to carry out the present invention; thus, the present invention is also industrially advantageous.

The amount of the emulsified functional composition of the present invention to be added to the composition, for which the functional substance is to be added, is not particularly limited insofar as it is within a general amount of a functional substance for adding a desired function to a functional product. For example, the amount of the emulsified functional composition of the present invention is 0.1 to 100 wt%, preferably 0.1 to 90 wt%, more preferably 0.1 to 80 wt%, per 100 wt% of the functional composition.

### Examples

The features and the effects of the present invention are more specifically explained below with reference to Examples and Experiment Examples. However, the present invention is not limited to the examples.

### Experiment Example 1

According to the prescriptions shown in Table 1, emulsified flavoring compositions (emulsified flavoring preparations) were prepared using various kinds of gum ghatti, gum arabic, or decaglycerol monooleate as an emulsifier (Reference Example 1, Examples 2 to 11, Comparative Examples 1 to 7) .

For each of the emulsified flavoring compositions thus prepared, the median particle diameter (µm) of the oil phase particles and the turbidity (1%E) immediately after the preparation, and the turbidity (1%E) after seven days of storage at 60°C were measured, thereby evaluating the preparation stability of the compositions. Further, beverages were prepared by incorporating these emulsified flavoring compositions in potable water, and the conditions (amounts of insoluble matter generated) of the beverages immediately after the preparation and after shaking were visually observed, thereby evaluating solubility in water and stability of the compositions.

### (1) Method for Preparing Emulsified Flavoring Composition

The oil-soluble materials (flavoring components), oil-based solvents (glycerin fatty acid ester (Salacos HG-8 (Nisshin Oillio Group, Ltd.)) and a medium-chain triglyceride [MCT] (O.D.O (Nisshin Oillio Group, Ltd.))), each in an amount shown in Table 1, were evenly mixed. The mixture was added to an aqueous emulsifier solution, which is obtained by dissolving an emulsifier (gum ghatti, gum arabic, modified starch, or decaglycerol monooleate) in ion exchange water together with citric acid. The resulting mixture was stirred and mixed. Then, emulsification was performed using a high-pressure homogenizer (15MR-8TA high-pressure homogenizer; APV Gaulin) under a pressure of 500 kg/cm², thereby preparing an emulsified flavoring composition (Reference Example 1, Examples 2 to 11, Comparative Examples 1 to 7).

The viscosity of gum ghatti was measured in the manner described below. Hereinafter, the "viscosity" of an emulsifier means a viscosity of a 15 wt% aqueous solution of the emulsifier measured under the following condition.

### ▪ Method for Measuring Viscosity

Each emulsifier was dissolved in water, thereby preparing a 15 wt% aqueous solution of the emulsifier. (If the emulsifier is not dissolvable in water, the emulsifier is dissolved by heating, and then the solution is cooled to 20°C.) The aqueous solution of the emulsifier was prepared to have a constant temperature (20°C), and the viscosity of the aqueous solution was measured by rotating it with a Brookfield rotational viscometer (Tokyo Keiki Inc., Model: BM) for a minute at a rotation speed of 30 rpm. For this measurement, Rotor No. 2 was used to measure a viscosity of 50 to 500 mPa·s, and Rotor No.3 was used to measure a viscosity of 500 to 4000 mPa·s.

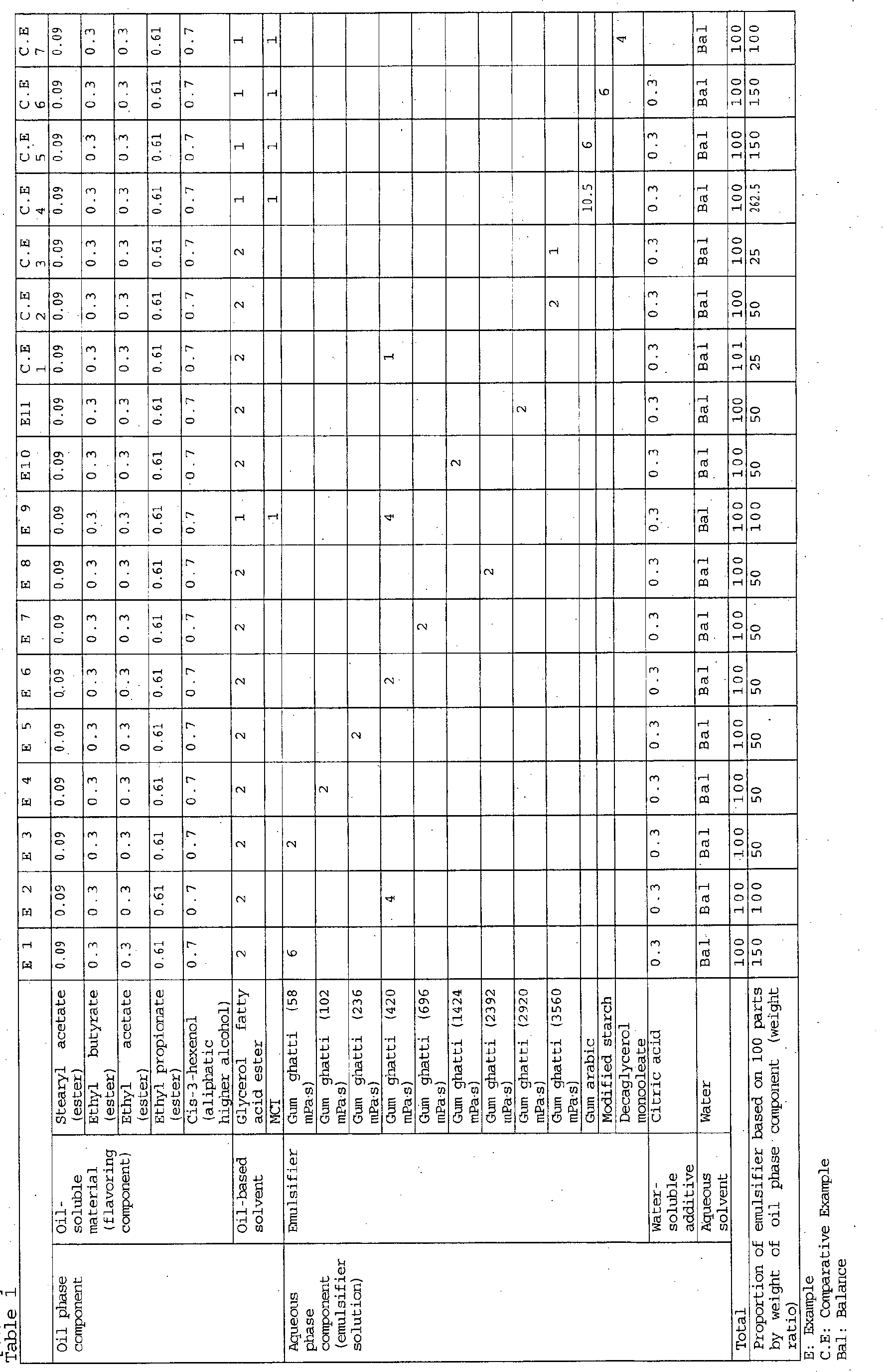

### (2) Evaluation

### (2-1) Evaluation of Emulsified Flavoring Composition (Emulsified Flavoring Preparation)

For each of the emulsified flavoring compositions immediately after the preparation, the diameter (median particle diameter: µm) of the emulsified particles in the oil phase was measured with a laser diffraction particle size analyzer (SALD-2100, Shimadzu Corporation).

Further, the emulsified flavoring compositions immediately after the preparation and the same emulsified flavoring compositions after seven days of storage at 60°C in a constant-temperature bath were individually diluted with ion exchange water so that the resulting solution had a concentration of 1 wt%. The absorbency of each aqueous solution at 720 nm was measured in comparison with ion exchange water, thereby evaluating the turbidity (1%E) of each solution. When the turbidity (1%E) of the solution immediately after the preparation was less than 0.5, it was evaluated that the composition has good solubility in water, and when the turbidity (1%E) of the solution immediately after the preparation was 0.5 or more, it was evaluated that the composition has poor solubility in water. Further, if the difference between the turbidity (1%E) of an emulsified flavoring composition immediately after the preparation, and the turbidity (1%E) of the same emulsified flavoring composition after seven days of storage at 60°C in a constant-temperature bath was less than ±50% of the initial value, it was evaluated that the composition has good emulsion stability. If the difference was ±50% or more of the initial value, it was evaluated that the composition has poor emulsion stability.

### (2-2) Evaluation of Beverage in Which Emulsified Flavoring Composition is Dissolved (Water Solubility and Storage Stability)

A beverage was prepared according to the method below using each emulsified flavoring composition prepared above.

### ▪ Prescription of beverage (unit: kg)

| | |
|---|---|
| High-fructose corn syrup (Brix 75°) | 10.7 |
| Citric acid (anhydrous) | 0.1 |
| Trisodium citrate | 0.015 |
| Emulsified flavoring preparation (Reference Example 1, Examples 2 to 11, Comparative Examples 1 to 7) | 0.5 |
| Ion exchange water | balance |
| Total | 100.000 |

### ▪ Method for Preparing Beverage

All the components of the beverage in the above prescription were dissolved in ion exchange water, and the solution was sterilized at 93°C. The solution was then contained in a 200-mL glass bottle and cooled.

Using beverages immediately after the preparation and beverages shaken for 30 minutes at normal temperature with a stroke of 2 cm, 140 strokes/min (beverages after shaking), the amounts of generation of suspended substances (amounts of generation of insoluble matter) were visually evaluated according to the following criteria.

### ▪ Amounts of Suspended Substances Generated (Amounts of Insoluble Matter Generated)

Suspended substances were not observed: -
A slight amount of suspended substance was observed: +
A small amount of suspended substances was observed: ++
A large amount of suspended substances was observed: +++

### (3) Results

Table 2 shows the results.

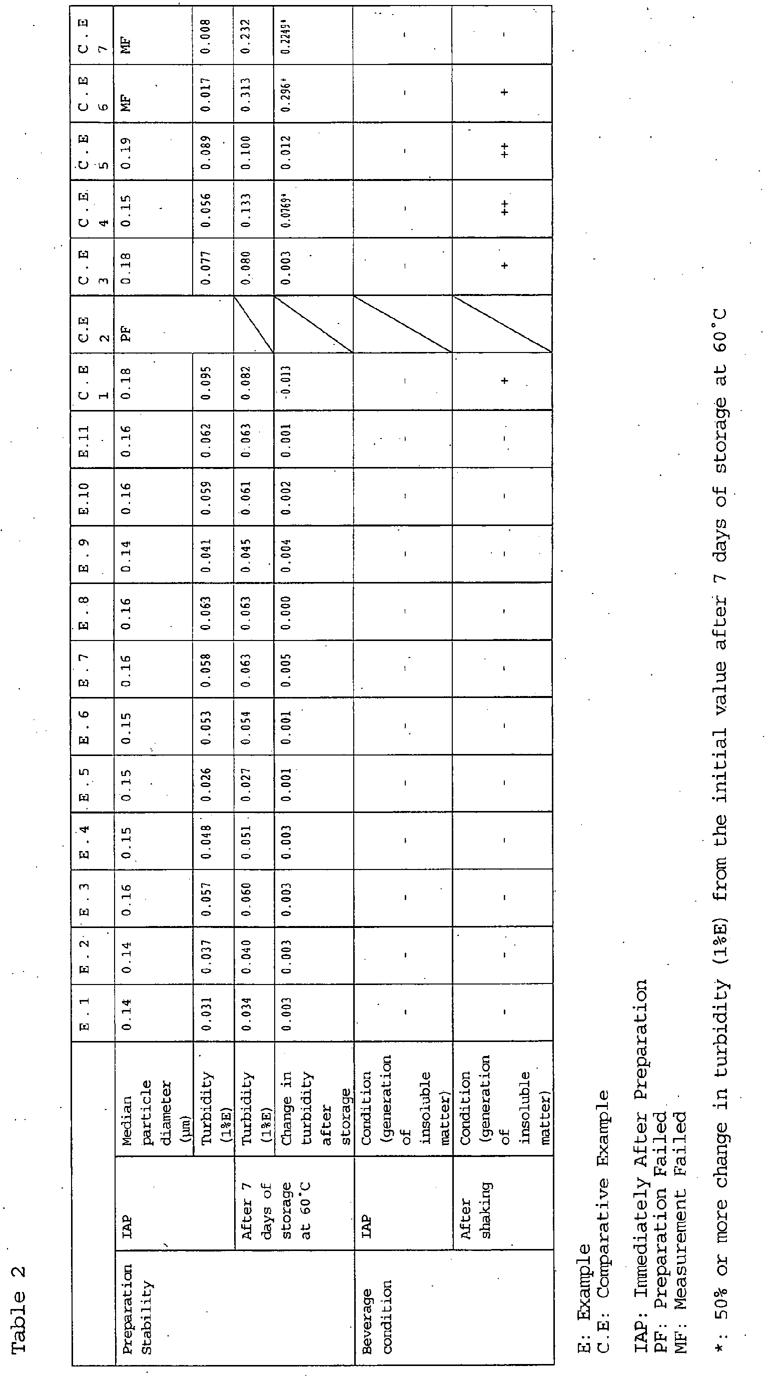

### ▪ Results

After seven days of storage at 60°C, no significant change was observed in the emulsified flavoring compositions (Examples 1 to 11) prepared by using, as an emulsifier, gum ghatti having a viscosity in a range of 50 to 3000 mPa·s in a proportion of more than 25 parts by weight based on 100 parts by weight, i.e., the total amount, of the oil phase component, compared with the emulsified flavoring composition (Comparative Example 4) prepared by using gum arabic, the emulsified flavoring composition (Comparative Example 6) prepared by using a modified starch, and the emulsified flavoring composition (Comparative Example 7) prepared by using-decaglycerol monooleate, which were also stored under the same conditions. Thus, these emulsified flavoring compositions containing gum ghatti had excellent emulsion stability.

When gum ghatti having a viscosity of more than 3000 mPa·s was used in a proportion of more than 25 parts by weight based on 100 parts by weight, i.e., the total amount, of the oil phase component, the viscosity of the emulsified flavoring composition significantly increased in the emulsification step, and the emulsified flavoring composition could not be prepared (Comparative Example 2).

Further, among these emulsified flavoring compositions, the generation of suspended substances after shaking was significantly suppressed in the beverages containing the emulsified flavoring compositions (Reference Example 1, Examples 2 to 11) prepared by using, as an emulsifier, gum ghatti having a viscosity in a range of 50 to 3000 mPa·s in a proportion of more than 25 parts by weight based on 100 parts by weight, i.e., the total amount, of the oil phase component, compared with the beverages containing the emulsified flavoring compositions (Comparative Examples 1 and 3) prepared by using gum ghatti in a proportion of 25 parts by weight or less based on 100 parts by weight, i.e., the total amount, of the oil phase component, or the beverage containing the emulsified flavoring composition (Comparative Examples 4 to 6) prepared by using gum arabic or a modified starch. Thus, these emulsified flavoring compositions containing gum ghatti in a proportion of more than 25 parts, by weight based on 100 parts by weight, i.e., the total amount, of the oil phase component had excellent stability.

### Experiment Example 2

According to the prescriptions shown in Table 3, emulsified colorant compositions (emulsified colorant preparations) (Examples 12 to 16, Comparative Examples 8 to 14) were prepared by using gum ghatti, gum arabic, β-pectin, or a modified starch, as an emulsifier. For the emulsified colorant compositions thus obtained, preparation stability was evaluated in the same manner as in Experiment Example 1. Further, beverages were prepared by using these emulsified colorant compositions, and the solubility in water and the storage stability of the beverages were evaluated by measuring the generation of suspended substances in the beverages immediately after the preparation and after shaking in the same manner as in Experiment Example 1.

### (1) Method for Preparing Emulsified Colorant Composition

The oil-soluble material (colorant component) and an oil-based solvent (medium-chain triglycerides [MCT]), each in an amount shown in Table 3, were evenly mixed. The mixture was then added to each of the aqueous emulsifier solutions obtained by dissolving the individual emulsifiers (gum ghatti, gum arabic, β-pectin, and a modified starch) in ion exchange water together with sodium benzoate and citric acid. The mixture was stirred and mixed. Then, emulsification was performed using a high-pressure homogenizer (APV Gaulin; 15MR-8TA high-pressure homogenizer) under a pressure of 500 kg/cm², thereby preparing emulsified colorant compositions (Examples 12 to 16, Comparative Examples 8 to 14).

The viscosity of gum ghatti was measured according to the method of Experiment Example 1.

## Claims

1. An emulsion composition prepared by emulsifying an oil phase component and an aqueous phase component using gum ghatti, wherein the emulsion composition comprises gum ghatti in a proportion of more than 25 parts by weight based on 100 parts by weight of the oil phase component; the gum ghatti has a viscosity of 50 to 3000 mPa·s, which is measured by preparing a 15 wt% aqueous solution of the gum ghatti and measuring its viscosity for 1 minute at 20°C and 30 rpm using a Brookfield viscometer; and
the absorbency (1%E) (cell width = 1 cm) of an aqueous solution obtained by diluting the emulsion composition immediately after preparation with water at a concentration of 1 wt% is less than 0.5, which is measured at a wavelength of 720 nm in comparison with ion exchange water, and wherein the proportion of gum ghatti in the aqueous phase component containing the gum ghatti is 1 to 6 wt% based on 100 wt% of the total amount of the aqueous phase component containing the gum ghatti.

2. The emulsion composition according to claim 1, wherein the oil phase component comprises at least one member selected from the group consisting of oil-soluble flavorings, oil-soluble colorants, and oil-soluble bioactive substances.

3. The emulsion composition according to claim 2, wherein the oil-soluble bioactive substance is at least one member selected from the group consisting of fat-soluble vitamins, docosahexaenoic acid, eicosapentaenoic acid, coenzyme Q₁₀, α-lipoic acids, α-linolenic acid, oil-soluble polyphenols, sesamin, phytosterols, and glycosyl ceramides.

4. The emulsion composition according to any one of claims 1 to 3, wherein the oil phase component comprises at least one member selected from the group consisting of glycerin fatty acid esters and medium-chain triglycerides.

5. The emulsion composition according to claim 4, wherein the glycerin fatty acid ester is a polyglycerin fatty acid ester in which 5 to 8 molecules of C₂₋₁₀ saturated fatty acid are bonded to polyglycerin having an average polymerization degree of 3 to 10 by ester bonds.

6. The emulsion composition according to claim 4 or 5, wherein the oil phase component further comprises lecithin.

7. The emulsion composition according to any one of claims 1 to 6, wherein the emulsion composition is at least one member selected from the group consisting of emulsified flavoring preparations, emulsified colorant preparations, and emulsified functional preparations.

8. The emulsion composition according to any one of claims 1 to 7, wherein the emulsion composition is an O/W type emulsion.

9. A method for preparing an emulsion composition comprising the step of emulsifying an oil phase component and an aqueous phase component using gum ghatti in a proportion of more than 25 parts by weight based on 100 parts by weight of the oil phase component,
wherein the gum ghatti has a viscosity of 50 to 3000 mPa·s, which is measured by preparing a 15 wt% aqueous solution of the gum ghatti and measuring its viscosity for 1 minute at 20°C and 30 rpm using a Brookfield viscometer, and
the absorbency (1%E) (cell width = 1 cm) of an aqueous solution obtained by diluting the emulsion composition immediately after preparation with water at a concentration of 1 wt% is less than 0.5, which is measured at a wavelength of 720 nm in comparison with ion exchange water, and wherein the proportion of gum ghatti in the aqueous phase component containing the gum ghatti is 1 to 6 wt% based on 100 wt% of the total amount of the aqueous phase component containing the gum ghatti.

10. The method according to claim 9, wherein the emulsion composition is at least one member selected from the group consisting of oil-based flavorings, oil-based colorants, and oil-based bioactive substances.

11. The method according to claim 9 or 10, wherein the oil phase component further comprises lecithin.

12. A method for preparing a composition selected from the group consisting of food, beverages, fragrances, cosmetics, pharmaceuticals and quasi-drugs, the method comprising the step of dissolving or dispersing the emulsion composition of any one of claims 1 to 8 in an aqueous solvent.

## Patentansprüche

1. Emulsionszusammensetzung, zubereitet durch Emulgieren einer Ölphasenkomponente und einer wässrigen Phasenkomponente unter Verwendung von Ghatti-Gummi, wobei die Emulsionszusammensetzung Ghatti-Gummi in einem Anteil von mehr als 25 Gewichtsteilen, bezogen auf 100 Gewichtsteile der Ölphasenkomponente, umfasst;
der Ghatti-Gummi eine Viskosität von 50 bis 3000 mPa·s aufweist, welche durch Zubereiten einer 15 Gew.-% wässrigen Lösung des Ghatti-Gummis und Messen seiner Viskosität für 1 Minute bei 20°C und 30 rpm unter Verwendung eines Brookfield-Viskosimeters gemessen wird; und
das Absorptionsvermögen (1%E) (Zellbreite = 1 cm) einer wässrigen Lösung, erhalten durch Verdünnen der Emulsionszusammensetzung unmittelbar nach Zubereitung mit Wasser, bei einer Konzentration von 1 Gew.-% weniger als 0,5 beträgt, welches bei einer Wellenlänge von 720 nm im Vergleich mit lonenaustauschwasser gemessen wird, und wobei der Anteil des Ghatti-Gummis in der wässrigen Phasenkomponente, enthaltend den Ghatti-Gummi, 1 bis 6 Gew.-% beträgt, bezogen auf 100 Gew.-% der Gesamtmenge der wässrigen Phasenkomponente, enthaltend den Ghatti-Gummi.

2. Emulsionszusammensetzung nach Anspruch 1, wobei die Ölphasenkomponente mindestens ein Mitglied, ausgewählt aus der Gruppe, bestehend aus öllöslichen Geschmacksstoffen, öllöslichen Farbstoffen und öllöslichen bioaktiven Substanzen, umfasst.

3. Emulsionszusammensetzung nach Anspruch 2, wobei die öllösliche bioaktive Substanz mindestens ein Mitglied, ausgewählt aus der Gruppe, bestehend aus fettlöslichen Vitaminen, Docosahexaensäure, Eicosapentaensäure, Coenzym Q₁₀, α-Liponsäuren, α-Linolensäure, öllöslichen Polyphenolen, Sesamin, Phytosterolen und Glycosylceramiden, ist.

4. Emulsionszusammensetzung nach einem der Ansprüche 1 bis 3, wobei die Ölphasenkomponente mindestens ein Mitglied, ausgewählt aus der Gruppe, bestehend aus Glycerinfettsäureestern und mittelkettigen Triglyceriden, umfasst.

5. Emulsionszusammensetzung nach Anspruch 4, wobei der Glycerinfettsäureester ein Polyglycerinfettsäureester ist, in welchem 5 bis 8 Moleküle von C₂₋₁₀-gesättigter Fettsäure an Polyglycerin mit einem gemittelten Polymerisationsgrad von 3 bis 10 durch Esterbindungen gebunden sind.

6. Emulsionszusammensetzung nach Anspruch 4 oder 5, wobei die Ölphasenkomponente weiter Lecithin umfasst.

7. Emulsionszusammensetzung nach einem der Ansprüche 1 bis 6, wobei die Emulsionszusammensetzung mindestens ein Mitglied, ausgewählt aus der Gruppe, bestehend aus emulgierten Geschmacksstoffzubereitungen, emulgierten Farbstoffzubereitungen und emulgierten funktionellen Zubereitungen, ist.

8. Emulsionszusammensetzung nach einem der Ansprüche 1 bis 7, wobei die Emulsionszusammensetzung eine Emulsion des O/W-Typs ist.

9. Verfahren zur Zubereitung einer Emulsionszusammensetzung, umfassend den Schritt des Emulgierens einer Ölphasenkomponente und einer wässrigen Phasenkomponente unter Verwendung von Ghatti-Gummi in einem Anteil von mehr als 25 Gewichtsteilen, bezogen auf 100 Gewichtsteile der Ölphasenkomponente,
wobei der Ghatti-Gummi eine Viskosität von 50 bis 3000 mPa·s aufweist, welche durch Zubereiten einer 15 Gew.-% wässrigen Lösung des Ghatti-Gummis und Messen seiner Viskosität für 1 Minute bei 20°C und 30 rpm unter Verwendung eines Brookfield-Viskosimeters gemessen wird, und
das Absorptionsvermögen (1%E) (Zellbreite = 1 cm) einer wässrigen Lösung, erhalten durch Verdünnen der Emulsionszusammensetzung unmittelbar nach Zubereitung mit Wasser, bei einer Konzentration von 1 Gew.-% weniger als 0,5 beträgt, welches bei einer Wellenlänge von 720 nm im Vergleich mit lonenaustauschwasser gemessen wird, und wobei der Anteil des Ghatti-Gummis in der wässrigen Phasenkomponente, enthaltend den Ghatti-Gummi, 1 bis 6 Gew.-% beträgt, bezogen auf 100 Gew.-% der Gesamtmenge der wässrigen Phasenkomponente, enthaltend den Ghatti-Gummi.

10. Verfahren nach Anspruch 9, wobei die Emulsionszusammensetzung mindestens ein Mitglied, ausgewählt aus der Gruppe, bestehend aus ölbasierten Geschmacksstoffen, ölbasierten Farbstoffen und ölbasierten bioaktiven Substanzen, ist.

11. Verfahren nach Anspruch 9 oder 10, wobei die Ölphasenkomponente weiter Lecithin umfasst.

12. Verfahren zur Zubereitung einer Zusammensetzung, ausgewählt aus der Gruppe, bestehend aus Lebensmitteln, Getränken, Düften, Kosmetika, Arzneimitteln und Quasimedikamenten, wobei das Verfahren den Schritt des Auflösens oder Dispergierens der Emulsionszusammensetzung nach einem der Ansprüche 1 bis 8 in einem wässrigen Lösungsmittel umfasst.

## Revendications

1. Composition d'émulsion préparée en émulsifiant un composant de phase huileuse et un composant de phase aqueuse à l'aide de ghatti, dans laquelle la composition d'émulsion comprend du ghatti dans une proportion de plus de 25 parties en poids par rapport à 100 parties en poids du composant de phase huileuse ; le ghatti a une viscosité de 50 à 3 000 mPa·s, qui est mesurée en préparant une solution aqueuse à 15 % en poids du ghatti et en mesurant sa viscosité pendant 1 minute à 20 °C et 30 tours par minute à l'aide d'un viscosimètre Brookfield ; et
le pouvoir absorbant (1 %E) (largeur de cellule = 1 cm) d'une solution aqueuse obtenue en diluant la composition d'émulsion immédiatement après préparation avec de l'eau à une concentration de 1 % en poids est inférieur à 0,5, qui est mesuré à une longueur d'onde de 720 nm en comparaison à de l'eau échangeuse d'ions, et dans laquelle la proportion de ghatti dans le composant de phase aqueuse contenant le ghatti est de 1 à 6 % en poids par rapport à 100 % en poids de la quantité totale du composant de phase aqueuse contenant le ghatti.

2. Composition d'émulsion selon la revendication 1, dans laquelle le composant de phase huileuse comprend au moins un élément choisi dans le groupe constitué d'aromatisants solubles dans l'huile, de colorants solubles dans l'huile, et de substances bioactives solubles dans l'huile.

3. Composition d'émulsion selon la revendication 2, dans laquelle la substance bioactive soluble dans l'huile est au moins un élément choisi dans le groupe constitué de vitamines liposolubles, d'acide docosahexanoïque, d'acide éicosapentaénoïque, de coenzyme Q₁₀, d'acides α-lipoïques, d'acide α-linolénique, de polyphénols solubles dans l'huile, de sésamine, de phytostérols, et de céramides de glycosyle.

4. Composition d'émulsion selon l'une quelconque des revendications 1 à 3, dans laquelle le composant de phase huileuse comprend au moins un élément choisi dans le groupe constitué d'esters d'acide gras de glycérine et de triglycérides à chaîne moyenne.

5. Composition d'émulsion selon la revendication 4, dans laquelle l'ester d'acide gras de glycérine est un ester d'acide gras de polyglycérine dans lequel 5 à 8 molécules d'acide gras saturé en C₂₋₁₀ sont liées à la polyglycérine ayant un degré de polymérisation moyen de 3 à 10 par liaisons ester.

6. Composition d'émulsion selon la revendication 4 ou 5, dans laquelle le composant de phase huileuse comprend en outre de la lécithine.

7. Composition d'émulsion selon l'une quelconque des revendications 1 à 6, dans laquelle la composition d'émulsion est au moins un élément choisi dans le groupe constitué de préparations d'aromatisants émulsifiées, de préparations de colorants émulsifiées, et de préparations fonctionnelles émulsifiées.

8. Composition d'émulsion selon l'une quelconque des revendications 1 à 7, dans laquelle la composition d'émulsion est une émulsion de type huile/eau.

9. Procédé de préparation d'une composition d'émulsion comprenant l'étape d'émulsification d'un composant de phase huileuse et d'un composant de phase aqueuse à l'aide de ghatti dans une proportion de plus de 25 parties en poids par rapport à 100 parties en poids du composant de phase huileuse,
dans lequel le ghatti a une viscosité de 50 à 3 000 mPa·s, qui est mesurée en préparant une solution aqueuse à 15 % en poids du ghatti et en mesurant sa viscosité pendant 1 minute à 20 °C et 30 tours par minute à l'aide d'un viscosimètre Brookfield, et le poids absorbant (1 %E) (largeur de cellule = 1 cm) d'une solution aqueuse obtenue en diluant la composition d'émulsion immédiatement après préparation avec de l'eau à une concentration de 1 % en poids est inférieur à 0,5, qui est mesuré à une longueur d'onde de 720 nm en comparaison à de l'eau échangeuse d'ions, et dans laquelle la proportion de ghatti dans le composant de phase aqueuse contenant le ghatti est de 1 à 6 % en poids par rapport à 100 % en poids de la quantité totale du composant de phase aqueuse contenant le ghatti.

10. Procédé selon la revendication 9, dans lequel la composition d'émulsion est au moins un élément choisi dans le groupe constitué d'aromatisants à base d'huile, de colorants à base d'huile, et de substances bioactives à base d'huile.

11. Procédé selon la revendication 9 ou 10, dans lequel le composant de phase huileuse comprend en outre de la lécithine.

12. Procédé de préparation d'une composition choisie dans le groupe constitué d'aliments, de boissons, de parfums, de produits cosmétiques, de produits pharmaceutiques et de produits parapharmaceutiques, le procédé comprenant l'étape de dissolution ou de dispersion de la composition d'émulsion de l'une quelconque des revendications 1 à 8 dans un solvant aqueux.
